## Europäisches Patentamt
## European Patent Office
## Office européen des brevets

(11) Publication number: **0 329 257**
**A2**

## EUROPEAN PATENT APPLICATION

(21) Application number: **89200394.8**

(22) Date of filing: **17.02.89**

(51) Int. Cl.⁴: **C12N 15/00 , A61K 39/015 , C12P 21/02**

(30) Priority: **19.02.88 US 157864**

(43) Date of publication of application:
**23.08.89 Bulletin 89/34**

(84) Designated Contracting States:
**AT BE CH DE ES FR GB GR IT LI LU NL SE**

(71) Applicant: **Microgenesys, Inc.**
**400 Frontage Road**
**West Haven New Haven Connecticut 06516(US)**

(72) Inventor: **Cochran, Mark A.**
**415 Brooksvale Avenue**
**Hamden Connecticut 06518(US)**
Inventor: **Smith, Gale E.**
**125 Michael Drive**
**Guilford Connecticut 06437(US)**
Inventor: **Volvovitz, Franklin**
**123 York Street**
**New Haven Connecticut 06511(US)**

(74) Representative: **Smulders, Theodorus A.H.J., Ir. et al**
**Vereenigde Octrooibureaux Nieuwe Parklaan 107**
**NL-2587 BP 's-Gravenhage(NL)**

(54) **Method for producing recombinant protein derived from the circumsporozoite gene of plasmodium falciparum.**

(57) The baculovirus-insect cell vector system is useful as a high efficiency eukaryotic cloning and expression method for the production of the recombinant proteins. A major application of this system is in the development of rDNA vaccines for important human and animal diseases. This system is also useful to express the circumsporozoite antigen of a parasite that causes malaria in humans, Plasmodium falciparum. The resulting recombinant protein consists of full-length circumsporozoite antigen. The results indicate high levels of expression into recombinant products which closely resemble the authentic product. Furthermore, it has been demonstrated to effect purification of the recombinant product to near homogeneity. The expression of this gene by the baculorvirus-insect cell vector system yields these desirable results because this gene is normally expressed by insects. Recombinant circumsporozoite proteins produced in this manner have application as ingredients for sub-unit vaccines to protect against malaria, and as an ingredient for malaria diagnosis.

FIG. 1

FRAGMENT MAP OF WT AND VECTOR DNA

Left-hand, promoter containing flank

Polyhedrin gene

Right-hand flank

MCS    MULTIPLE CLONING SITE

# METHOD FOR PRODUCING RECOMBINANT PROTEIN DERIVED FROM THE CIRCUMSPOROZOITE GENE OF PLASMODIUM FALCIPARUM

## BACKGROUND OF THE INVENTION

The production of effective vaccines for any disease depends on the ability to manufacture large quantities of a safe antigen that stimulates protective immunity when injected into human beings. Recombinant DNA technology presents the best option to antigen production because of its perceived ability to produce large quantities of safe and economical immunogens. The choice of which recombinant systems (bacterial, yeast, or other eukaryotic cells) to use involves considerations pertaining to the properties of the selected foreign gene and its product. The candidate gene product may require specific processing by glycosylation and cleavage. The recombinant system should be capable of processing the gene product in a manner which would yield a recombinant product which is similar to the authentic product.

Many important diseases of man involve infectious agents which are biologically transmitted between susceptible vertebrate hosts through the bite of a hemophagous arthropod (insect). Examples of such diseases include malaria, yellow fever, dengue fever, Japanese encelphalitis, leishmaniasis and trypanosomiasis. Generally, the infectious agents have the capacity to multiply in the tissues of the susceptible arthropods without producing any apparent diseases or damage. In nature, the vertebrate-anthropod-vertebrate cycle is maintained by the vector, which develops a lifelong infection through ingestion of vertebrate blood from an infected (viremic) host.

Baculoviruses can be used as high efficiency eukaryotic cloning and expression vectors for the production of recombinant proteins in cultured insect cells. Baculoviruses have more than the necessary requirements for use as eukaryotic cloning and expression vectors. Baculoviruses are safe by virtue of their narrow host range which is restricted to arthropods; they are capable of accomodating very large amounts of exogenous DNA; their structural proteins are post-translationally modified; their cells culture systems are safe, non-transformed, and efficient; and they possess the high efficiency polyhedrin promoter, which is more active than any other known promoter in virus-infected eukaryotic cells.

The baculovirus system also offers strong advantages in the production of polypeptides for the use in diagnostic procedures. Many humans and animals generally possess antibodies which react with bacterial yeast and heterologous histocompatibility antigens. The presence of these antibodies diminishes the reliability of diagnostic procedures due to false positive reactions with contaminating bacterial, yeast and mammalian proteins found in preparations produced in the respective expression systems. Humans and animals most likely do not have prior history of immunologic exposure to lepidoteran cell or baculovirus antigens. The lepidoteran cells and baculovirus employed in production of recombinant proteins of this invention are therefore more likely not to have the problem of contaminating antigens that are recognized by antibody normally present in humans or animals. Therefore, it is doubtful that contaminants of lepidopteran cell or baculovirus original would lead to false positive reactions in diagnostic procedures employing recombinant antigens produced in this system. Accordingly, the baculovirus-insect cell expression system presents an attractive and viable opportunity to the production of effective immunogens which are normally expressed by insects.

Malaria is a serious health problem in large areas of the world, affecting about 300 million people annually resulting in two to four million deaths. The disease causes periodic outbreaks of chills and fever, and can lead to coma and death in less hardy victims. In tropical Africa, almost all children over a year old suffer from malaria, and at least a million of them die from it annually. Carried by DDT-resistant strains of mosquitoes, newly evolved, drug-resistant strains of malaria parasites are on the rise. In India, for example, the incidence of malaria increased from 60,000 cases in 1962 to more than 40 million cases a year today.

There are four species of human malaria (reviewed in Miller et al., 1986): Plasmodium falciparum, a major human pathogen responsible for all malaria-related deaths; P. vivax, also widespread and causing considerable morbidity; and the less prevalent species P. ovale and P. malariae. There are numerous stages in the parasitic life cycle of the malaria organism, each morphologically and antigenically distinct. The cycle in the mosquito vector (female Anopheles mosquitoes) involves ingestion from malaria infected humans of red cell parasites which have differentiated into gametocytes. Fertilization of the gametocyte occurs rapidly in the insect gut. Within 24 hours zygotes transform into ookinetes which penetrate the midgut to form oocyts and later from sporozoites. It is the sporosoites which are the infectious agents when inoculated into the human bloodstream where they rapidly invade hepatic cells.

Plasmodium sporozoites are coated with a highly immunogenic circumsporozoites (CS) protein. The CS protein has been shown to induce protective immunity and has been investigated for its potential as a component in a malaria vaccine. The structure of the CS proteins of all malaria species includes a central domain of tandemly repeated amino acid composition of this repetitive region differs among the various malaria species (Nussenzweig and Nussensweig, 1985; Samtoro et al, 1983). This epitope in the CS protein of P. falciparum is a repetitive four amino acid sequence: asparagine-alanine-asparagine-proline, or NANP (Dame et al, 1984; Enea et al, 1984).

There is a large and well established body of knowledge concerning the development of a vaccine to protect against malaria (reviews in Miller, 1985; Miller et al, 1986; Ravetch et al, 1985). Protection against malaria has been demostrated to correlate with antibodies directed against the CS protein. Monoclonal antibodies to the NANP epitope have been shown to neutralize sporozoite infectivity in vitro. Recently, synthetic NANP peptides conjugated to protein carriers generate specific antisporozoite antibodies in animals and humans.

There has been limited success in conferring protection in human volunteers. Two independent trials have been conducted with similar candidate vaccines. The vaccine used in the clinical of Herrington et al (1987) was a synthetic peptide, (NANP)₃ conjugated to tetanus toxoid and the trial run by Ballou et al (1987) used a recombinant protein consisting of 32 of the NANP units and a short 32 amino acid C-terminal portion derived from the plasmid vector. Each of these vaccines contained the linear epitopes defined by the repeated amino acids sequences. In each study, the volunteer who had the highest antibody titre was protected from malaria following challenge with sporozoites inoculated by infected mosquitoes.

When taken together these clinical studies suggest that a CSA based sub-unit vaccine will indue protective antibodies in humans.. However, in order to manufacture an efficient malaria vaccine, the following must be met.

1. An effective candidate vaccine must induce high and sustained antibody titres in order to achieve antibody-mediated protection (Miller et al, 1986). Constant high levels of antibody could be maintained by subsequent boosts following natural exposure to P. falciparum sporozoites.

2. Formation of anti-sporozoite antibodies requires T-cell help which should preferably be derived from the sporozoite itself (Good et al, 1987). Mouse experiments have shown that the sporozoite protein contains T-cell sites both inside and outside the repetitive region which are restricted by the major histocompatibility system (MHC Class II) of the vaccine recipient (Good et al, 1986; 1987; Del Giudice, 1986).

3. Additional antibody independent T-cell activating sites may be required for direct T-cell mediated inhibition of parasite development in the liver. This has been shown to be the case with a murine malaria model (Egan et al, 1987) using irradiated sporozoites contrasted with subunit vaccines.

The full-length recombinant protein produced from recombinant virus infected insect cells as described here will serve as a more potent immunogen and contain many more important B and T-cell epitopes than in previously tested candidate vaccines.

The practices of this invention are set forth hereinafter with reference to the drawings wherein:

Fig. 1 is a restriction enzyme fragment map of wild-type (wt) and vector DNA;

Fig. 2 shows the construction of recombinant vector p3691;

Fig. 3 shows the construction of recombinant vector p4148;

Fig. 4 illustrates sequences relevant to plasmid constructions; and wherein

Fig. 5 shows the make-up of the circumsporozoite protein of P. falciparum.

## BRIEF DESCRIPTION OF THE INVENTION

The baculovirus-insect cell vector system has been used to express the circumsporozoite antigen of a parasite that causes malaria in humans, Plasmodium falciparum. The resulting recombinant protein consists of full-length circumsporozoite antigen.

## DETAILED DESCRIPTION OF THE INVENTION

METHODS

The baculovirus Autographa californica polyhedrosis virus (AcMNPV) was used. The promoter sequence used for expression is that of the polyhedrin (occ) gene derived from this virus. AcMNPV and recombinant virus stocks were maintained in Spodoptera frugiperda (fall armyworm) cells. The recombinant was constructed such that the Pn gene was deleted which renders the recombinant virus occ⁻. This strategy allows easy identification of recombinant viruses simply by plaque morphology.

## Construction of Insertion Vectors

Cloning and expression of foreign protein coding sequence in a baculovirus vector requires that the coding sequence be aligned with the polyhedrin promoter and upstream sequences and on the other side with truncated polyhedrin coding sequences such that homologous recombination with the baculovirus genome results in transfer of the foreign coding sequence aligned with the polyhedrin promoter and an inactive polyhedrin gene.

Accordingly, insertion vectors were designed for use in these gene constructions.

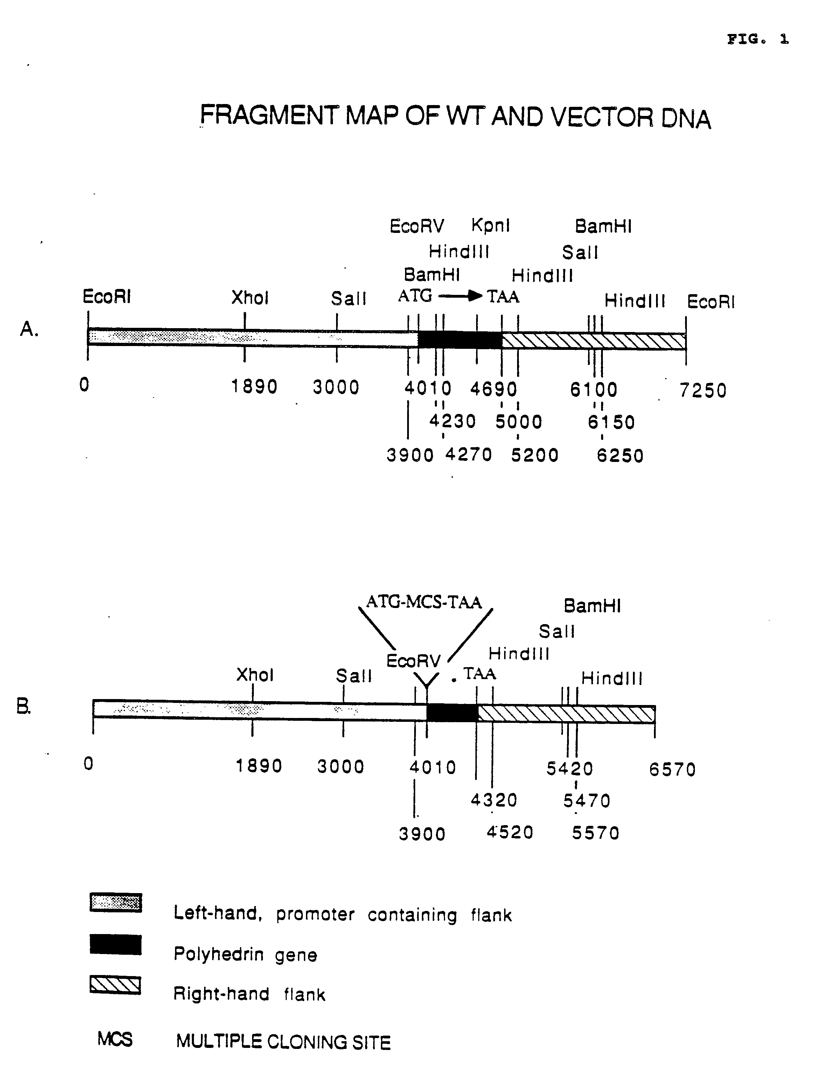

As identified hereinabove, Fig. 1 is a restriction enzyme fragment map of wild-type (wt) and vector DNA. At A of Fig.1 is the linear map of the EcoRI-I fragment of AcMNPV DNA. Polyhedrin encoding region is shown as a blackened area with the direction of transcription shown by the arrow running from the ATG translational initiation codon to the TAA translational termination codon. The left-hand flanking sequence contains the polyhedrin transcriptional termination signals and is shown as a hatched area.

In Fig. 1 at B, is shown the linear map of engineered EcoRI fragment to serve in insertion vectors. The fragment was engineered in the following manner: a synthetic oligomer (made up of sequences from the EcoRV site to the ATG and a region containing multiple unique restriction sites, followed by a universal stop codon sequence, and ending with KpnI compatible tail) was cloned to replace sequences lying between the EcoRV and KpnI sites. This produced a deletion of the N-terminal half of the polyhedrin gene and an insertion containing cloning sites immediately downstream from the authentic ATG translational initiation condon.

Accordingly, as shown in Fig. 1, the EcoRI-fragment (which contains the polyhedrin gene and its regulatory 3lements, Fig. 1A) was engineered to contain a multiple cloning site immediately downstream of the ATG translational initiation codon of the polyhedrin gene (Fig. 1B). In addition, the multiple cloning region was followed by a series of TAA translational termination codons. Furthermore, the N-terminal portion of the polyhedrin coding region was deleted. Each insertion vector described below was designated to supply the ATGH translational initiating codon. Insertion of foreign sequences into these vectors must be engineered such that the translational frame established by the initiating codon is maintained correctly through the foreign sequences.

Insertion vector pMGS3 + 1 was used for expression of Pf-CSA. The vector consists of the following structural features: 4000 bp of sequence upstream from the ATG initiating codon of the polyhedrin gene; a polylinker introduced by site directed mutagenesis, which consists of an ATG initiating codon at the exact position of the corresponding polyhedrin codon, and restriction sites SmaI, KpnI, BglII and a universal stop codon segment; 1700 bp of sequence extending from the KpnI restriction site (which is internal to the polyhedrin gene) through to the terminal EcoRI restriction site of the EcoRI-clone.

Insertion vector pMGS-mal was designed to yield the precise N-terminal amino acid sequence of Pf-CSA. The polylinker segment consisted of the first 5 amino acid codons of Pf-CSA followed by NruI and KpnI restriction sites.

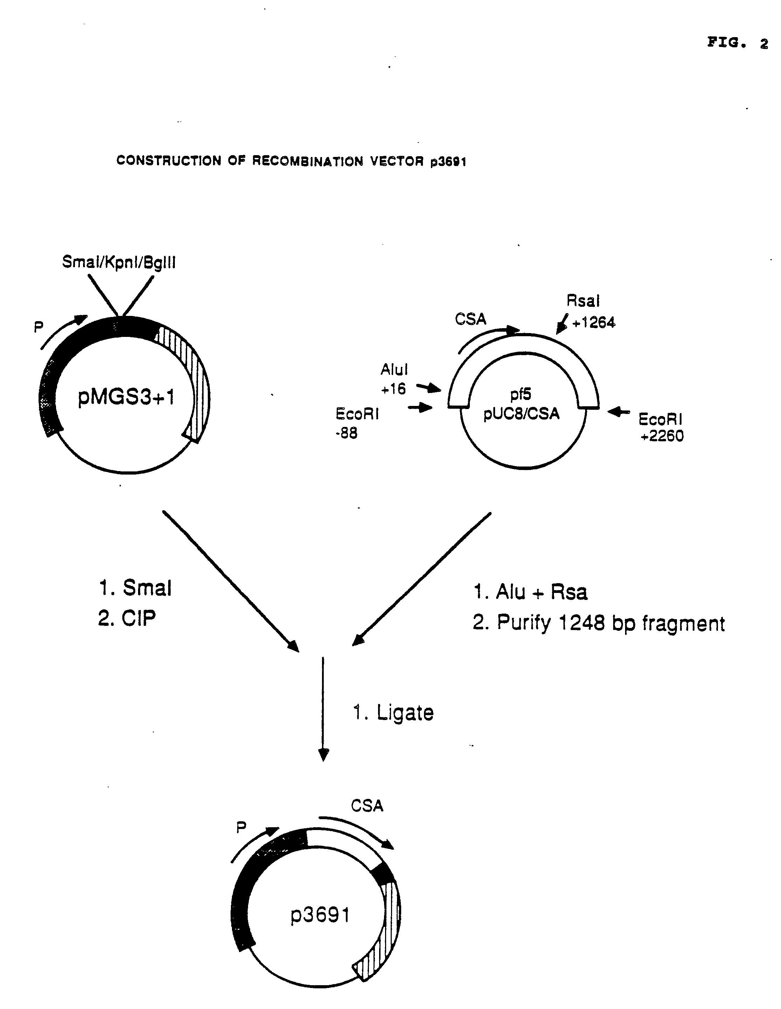

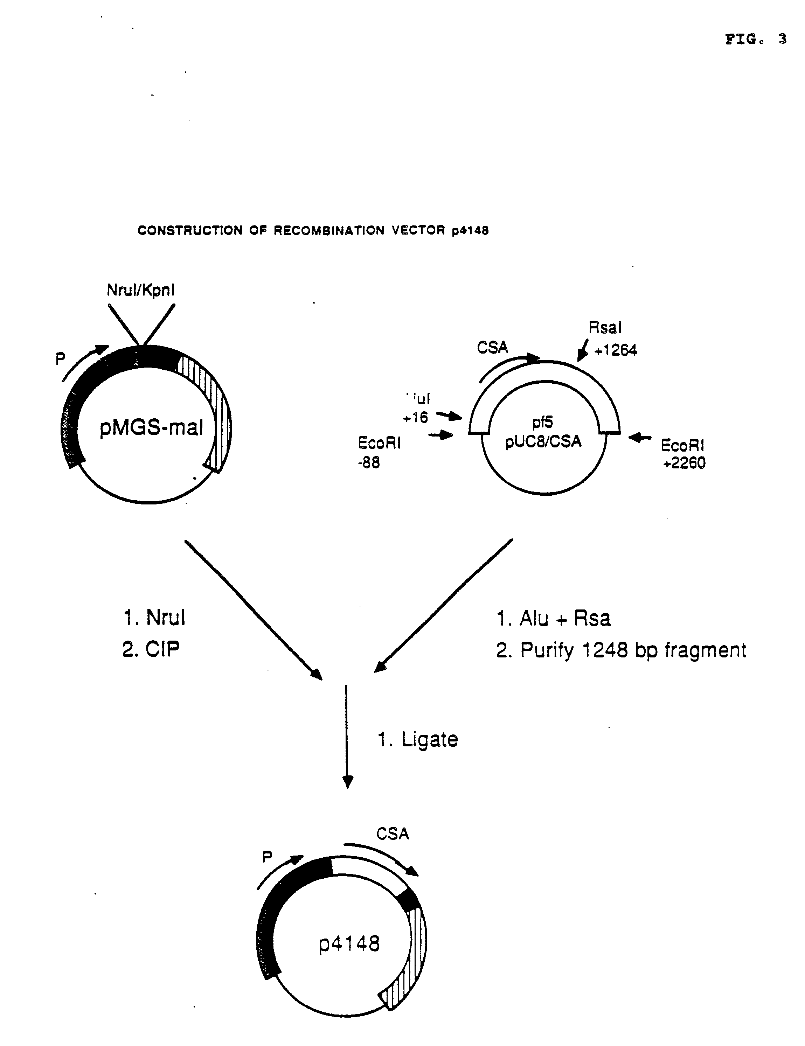

The cloning strategy for Pf-CSA is shown in Figs. 2 and 3. Fig. 2 shows the construction of the recombinant vector p3691. The plasmid maps show the isolation of the CS protein gene as a 1248 base pair AluI/RsaI fragment from plasmid pf5. This blunt-ended fragment was cloned into the SmaI site of vector pMGS3 + 1 to produce recombinant p3691 where the direction of transcription from the polyhedrin promoter (P) reads in-frame through the CS protein gene. Arrows indicate direction of transcription.

Fig. 3, as indicated hereinabove, shows the construction of recombinant vector p4148. The plasmid maps show the isolation of the CS protein gene as a 1248 base pair AluI/RsaI fragment from plasmid pf5. This blunt-ended fragment was cloned into the NruI site of vector pMGS-mal to produce recombinant p4148 where the direction of transcription from the polyhedrin promoter (P) reads in-frame through the CS protein gene. Arrows indicate the direction of translation.

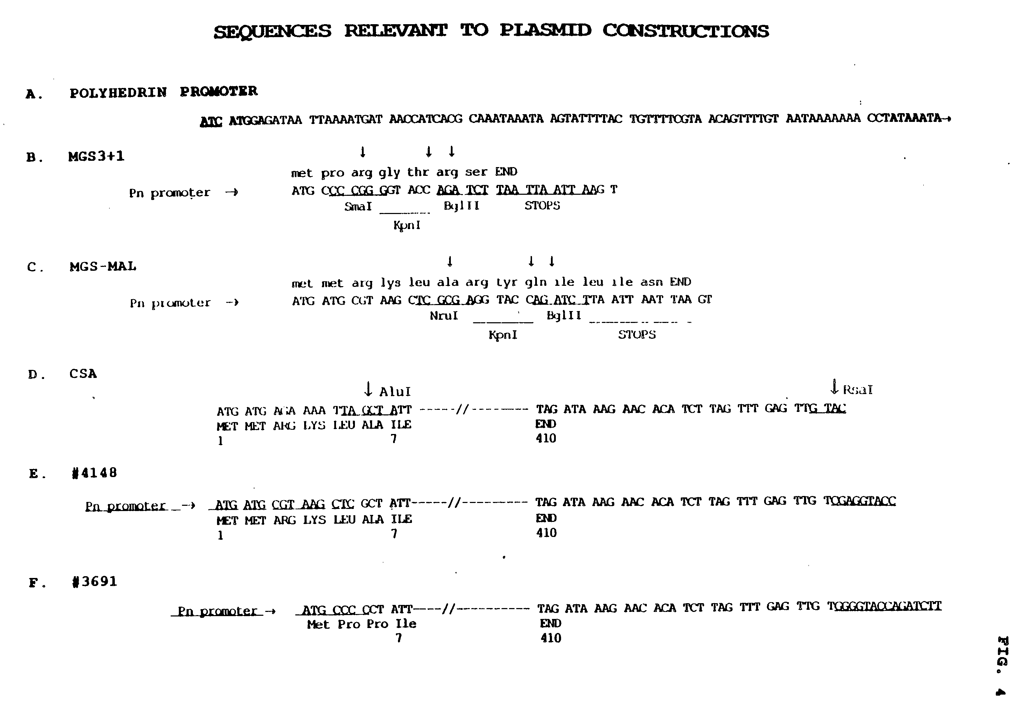

The sequences relevant to these plasmid constructions is presented in Fig. 4.

In Fig. 4 are shown sequences relevant to plasmid constructions. At A of Fig. 4, is shown the polyhedrin (Pn) promoter containing sequence from the EcoRV site to the ATG proximal base. This sequence matches that which normally lies upstream from the polyhedrin gene. It has been demonstrated that the polyhedrin promoter activity lies within this 103 base pair fragment. This sequence was synthesized for construction of the MGS vectors. At B of Fig. 4, is shown vector MGS3 + 1 which contains the ATG translational initiation codon at the same position as found in the polyhedrin gene followed by a polylinker that includes restriction sites for SmaI, KpnI, Bg1II and a universal stop codon segment. Restriction sites are indicated by underlined sequences and arrows showing the points of cleavage. Amino acid condons are shown as established by the ATG codon.

At C of Fig. 4, it is shown vector MGS-mal contains the ATG translational initiation codon at the same position as found in the polyhedrin gene followed by a polylinker that includes a sequence that codes for the first six amino acids of the authentic CSA gene, but uses the preferred codon usage as defined by the polyhedrin gene. This sequence also includes an Nrul restriction site which was designed for ligation to the Alul end of the CSA gene fragment. This followed by restriction sites for KpNl, Bg1ll and a universal stop codon segment. Restriction sites are indicated by underlined sequences and arrows showing the points of cleavage. Amino acid codons are shown as established by the ATG codon.

Further, at D of Fig. 4 is shown the CSA gene sequence of the N-terminal end including the ATG transla tional initiation codon and the Alul restriction enzyme site; and the C-terminal end including the translational termination codon and the Rsal restriction enzyme site. At E of Fig. 4 is shown the structure of recombinant 4148 which resulted from ligation of the CSA Alul/Rsal fragment to the Nrul site of pMGS-mal. Sequences contributed by the vector are underlined and at F of Fig. 4 is shown the structure of recombinant 3691 which resulted from ligation of the CSA Alu/Rsal fragment to the Smal site of pMGS3 + 1. Sequences contributed by the vector are underlined.

Recombinant v3691 consists of the CSA gene isolated as an Alul-Rsal fragment (1233 base pairs which spans from the sixth codon of the N-terminus through to the end of the gene) inserted downstream from the polyhedrin promoter as supplied by vector pMGS3 + 1. The resulting recombinant product is predicted to have vector derived amino acids-Met/Pro/Pro at the N-termini reading into the seventh amino acid (Ile) of authentic CS antigen.

Recombinant v4148 consists of the same CSA gene-containing fragment inserted downstream from the Pn promoter in vector pMGS-mal. this is a specialized vector which was designed to provide authentic N-terminal amino acids.

Expression Results

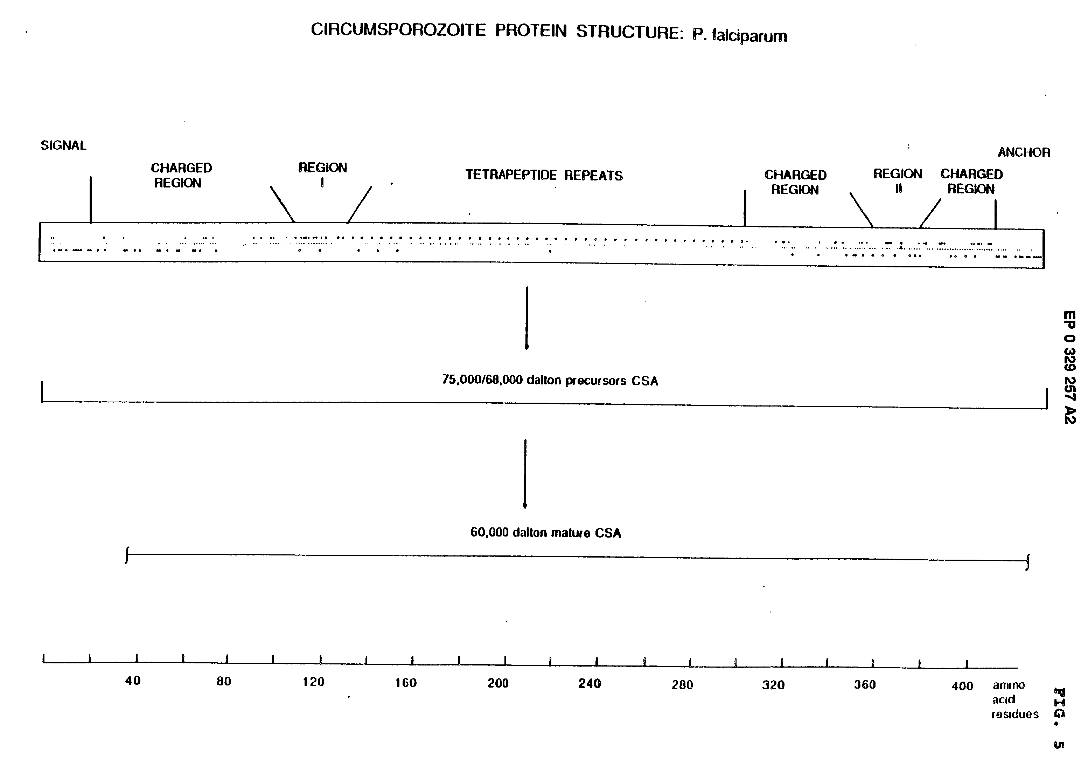

The structure of P. falciparum CS protein is illustrated in Fig. 5 which shows the structure of the circumsporozoite protein of P. falciparum. The figure is adapted from Dame et al (1984) and Nussenzweig and Nussenzweig (1985). As predicted by the nucleotide sequence (Dame et al, 1984), each amino acid is represented as a dot. Amino acids carrying a charge at physiological pH are indicated above the dots (Asp or Glu as - ; Arg or Lys as + ). The hydrophobic amino acids Ile, Leu, Phe Trp, Tyr or Val are indicated by the symbol below the dots. Pro and Cys are designated as P and C, respectively. Region I and Region II indicate amino acid stretches which are conserved between the CSA of P. falciparum and P. knowlesi. the approximate sizes of precursor and mature form of the CS polypeptide are indicated.

Expression of authentic CS protein is through two precursors (intracellular) forms that generate into mature CS protein molecules by sequential removal of two small basic peptides (Cochrane et al, 1982; Santoro et al, 1983, Vergera et al, 1985). The mature membrane form of the CS molecule is 50-100 amino acids shoerter than the precursor. The cleavage points have yet to be defined, however, and could be at either the N-terminus, or C-terminus, or from both the N- and C- termini.

Expression of recombinants v4148 and v 3691 in infected insect cells generates high molecular weight precursors which are sequentially cleaved to produce abundant quantities of mature recombinant CS protein.

The recombinant expression vectors p3691 and p4148 prepared in accordance with the practices of this invention are useful for the preparation of recombinant circumsporozoite antigen of the parasite Plasmodium falciparum which causes malaria in humans, as indicated hereinabove. Further, also, as indicated hereinabove, the resulting produced recombinant circumsporozoite antigen protein is usefully employed in the practices of this invention in a vaccine against malaria and in compositions for the diagnosis of malaria. When em ployed as a vaccine, the produced recombinant circumsporozoite antigen protein, such as may be produced in a baculovirus insect cell vector system in accordance with the practices of this invention is purified and prepared along with a suitable adjuvant and/or carrier in an effective amount to produce a vaccine composition which is, upon admnistration to a human, would serve to produce protection against or immunity to malaria infection. Also, a composition containing the produced circumsporozoite antigen, upon administration, such as by injection into a human, could be useful for the treatment of malaria. Further, the resulting produced circumsporozoite antigen protein could be used in a diagnostic composition for the diagnosis of malaria in humans.

In co-pending, co-assigned patent application Ser. No.     , filed February 3, 1988 entitled "Vaccine Containing Polypeptides Derived from the Envelope Gene of Human Immunodeficiency Virus Type 1", there is described a technique for utilizing a baculovirus insect cell vector system for the production of polypeptides which serve as AIDS antigen and for the recovery and purification of such polypeptides, after expression therein, from the baculovirus insect cell vector system. The disclosures of this application insofar as the disclosures are applicable to the practices of this invention, particularly for purification of vaccine compositions and the like, are herein incorporated and made part of this disclosure.

Also, in co-pending, co-assigned patent application Serial No. 006,880 filed January 27, 1987 entitled "HIV gp160 AIDS Virus Envelope Protein-Containing Strips and Use Thereof for Detection of Aids Antibody", there are disclosed techniques and compositions for the utlization of antigen proteins or diagnostic agents, which techniques are also applicable in the practices of this invention involving the utilization of the circumsporozoite antigen proteins for the diagnosis of malaria in humans. The disclosures of this application Serial No. 006,880 accordingly are also herein incorporated and made part of this disclosure.

Finally, in co-pending, co-assigned application Serial No. 920,197 filed October 16, 1986 entitled "Polypeptides Derived from the Envelope Gene of Human Immunodeficiency Virus in Recombinant Baculovirus Infected Insect Cells", there are disclosed techniques involving the utilization of baculovirus genes and AIDS virus genes and the like for utilization in a baculovirus-insect cell vector system for the production of polypeptides useful as antigens and other uses. The disclosures of this application Serial No. 920,197 related to the practices of this invention are herein also incorporated and made part of this disclosure.

As indicated hereinabove, the techniques of this invention are not only useful for the preparation of antigen material, as described herein, for the preparation of a vaccine useful against malaria in humans, but also useful for the detection of malaria antibodies in humans, i.e. as a diagnostic agent. Further, the recombinant circumsporozoite antigen proteins of this invention are useful not only as a vaccine and as a diagnostic element but are also useful in the treatment of malaria in humans for increasing malaria antibody content and activity in humans against malaria.

Further, the disclosures of all the publication references cited herein are herein incorporated and made part of this disclosure.

Following is a complete identification of the citation references mentioned hereinabove.

Ballou, W.R, J. Rothbard, R.A. Wirtz, et al.. 1985. Immunogenicity of synthetic peptides from circumsporozoite protein of Plasmodium falciparum. Science. 228:996-999.

Ballou, W.R, J.A. Sherwood, F.A. Neva, et al. 1987. Safety and efficacy of a recombinant DNA Plasmodium falciparum sporozoite vaccine. The Lancet. 1:1277-1281.

Cochrane, A.H., F. Santoro, V. Nussenzweig, R.W. Gwadz and R.S Nussensweig. 1982. Proc. Natl. Acad. Sci. USA 79:5651-5655.

Dame, J.B., J.L. Williams T.F. McCutchan, et al. 1984. Structure of the gene encoding the immunodominant surface antigen on the sporozoite of the human malaria parasite Plasmodium falciparum. Science. 225:593-599.

Del Giudice, G., J.A. Cooper, J. Merino et al. 1986. The antibody response. WHO Technical Report Series, No. 735. 1986. Expert Committee on Malaria: Eighteenth report.

Egan, J.E., J.L. Weber, W.R. Ballou et al. 1987. Efficacy of murine malaria sporozoite vaccines: Implications for human vaccine development. Science 236: 453-456.

Enea,V., J. Ellis, F. Zavala, et al. 1984. DNA cloning of Plasmodium falciparum circumsporozoite gene: amino acid sequence of repetitive epitope. Science. 255:628-630.

Galinski M.R., D.E. Arnot, A.H. Cochrane, et al. 1987. The circumsporozoite gene of the Plasmodium cynomolgi complex. Cell. 48:311-319.

Good, M.F., J.A. Berzofsky, W.L. Maloy et al. 1986. Genetic control of the immune responses in mice to Plasmodium falciparum sporozoite vaccine. Widespread nonresponsiveness to single malaria T epitope in highly repetive vaccine. J. Exp Med 164:655-60.

Good, M.F., W.L. Maloy, M.W. Lunde, et al. 1987. Construction of synthetic immunogen: use of new T-helper epitope on malaria circum sporite protein. Science 235:1059-1062.

Herrington, D.A., D.F. Clyde, G. Losonsky, et al. 1987. Safety and immunogenicity in man of a synthetic peptide malaria vaccine against Plasmodium falciparum sporozoites. Nature. 328:257-259.

Mazier, D., S. Mellouk, R.L. Beaudoin, et al. 1986. Effect of antibodies to recombinant and synthetic peptides on P. falciparum sporozoites in vitro. Science. 231:156-159.

Miller, L.H. 1985. Research toward a malaria vaccine: A critical review. Vaccine 85. ED R.A. Learner, R.M. Chanock and F. Brown. Cold Spring Laboratory, New York. pp 1-5.

Miller L.H., R.J. Howard, R. Carter, et al. 1986. Research toward malaria vaccines. Science. 234:1349-1356.

Nussenzweig, V., and R.S.Nussenzweig. 1985. Circumsporozoite proteins of malaria parasites. Cell. 42:401-403.

Ravetch, J.V., J. Young and G. Poste. 1985. Molecular genetic strategies for the development of antimalarial vaccines. Bio/Technology. 3:729-740.

Santoro, F., A.H Cochrane, V. Nussenweig, et al. 1983. Structural similarites among the protective antigens of sporozoites from different species of malaria parasites. J. Biol. Chem. 258:3341-3345.

Vergera, U., R. Gwadz, D. Schlesinger, V. Nussenzweig and A. Ferreira. 1985. Multiple non-repeated epitopes on the circumsporozoite protein of P. knowlesi. Mol. Biochem. Parasit. 14: 283-292.

Young, J.F., W.T. Hockmeyer, M. Gross, et al.

Expression of *Plasmodium falciparum* circumsporozoite proteins in *Escherichia coli* for potential use in a human malaria vaccine. Science. 228:958-962.

**Claims**

1. Recombinant circumsporozoite antigen protein of the parasite Plasmodium falciparum which causes malaria in humans.

2. A vaccine composition consisting essentially of the recombinant circumsporozoite antigen of the parasite Plasmodium falciparum in accordance with Claim 1 and a carrier therefor.

3. Recombinant circumsporozoite antigen protein in accordance with Claim 1 expressed in and recovered from a baculovirus-insect cell vector system.

4. A vaccine composition consisting essentially of the recombinant circumsporozoite antigen protein of Claim 3.

5. A composition useful for the diagnosis of malaria in humans comprising the recombinant circumsporozoite antigen protein of Claim 1 and a carrier therefor.

6. Full length recombinant circumsporozoite antigen protein in accordance with Claim 1.

7. Recombinant expression vector p3691.

8. Recombinant expression vector p4148.

9. A method of producing the recombinant circumsporozoite antigen protein in accordance with Claim 1 which comprises employing the expression vector p3691 in a baculovirus-insect cell vector system for the expression and production of the recombinant circumsporozoite antigen protein in accordance with Claim 1.

10. A method of producing the recombinant circumsporozoite antigen protein in accordance with Claim 1 which comprises employing the expression vector p4148 in a baculovirus-insect cell vector system for the expression and production of the recombinant circumsporozoite antigen protein in accordance with Claim 1.

11. A baculovirus-insect cell vector system comprising the recombinant expression vector of Claim 7.

12. A baculovirus-insect cell vector system comprising the recombinant expression vector of Claim 8.

13. A method of treating malaria in humans which comprises administering an effective amount of the antigen protein in accordance with Claim 1.

14. A method of diagnosing malaria in humans which comprises adding to the serum of the human an effective amount of the antigen protein of Claim 1.

FIG. 1

# FRAGMENT MAP OF WT AND VECTOR DNA

Legend:

- Left-hand, promoter containing flank
- Polyhedrin gene
- Right-hand flank

MCS    MULTIPLE CLONING SITE

FIG. 2

CONSTRUCTION OF RECOMBINATION VECTOR p3691

FIG. 3

CONSTRUCTION OF RECOMBINATION VECTOR p4148

# SEQUENCES RELEVANT TO PLASMID CONSTRUCTIONS

**A.  POLYHEDRIN PROMOTER**

ATC ATGGAGATAA TTAAAATGAT AACCATCACG CAAATAAATA AGTATTTTAC TGTTTTCGTA ACAGTTTTGT AATAAAAAAA CCTATAAATA→

**B.  MGS3+1**

             ↓    ↓ ↓

           met pro arg gly thr arg ser END

Pn promoter →   ATG CCC CGG GGT ACC AGA TCT TAA TTA ATT AAG T

            SmaI  ———— BglII   STOPS

              KpnI

**C.  MGS-MAL**

               ↓     ↓ ↓

           met met arg lys leu ala arg tyr gln ile leu ile asn END

Pn promoter →   ATG ATG CGT AAG CTC GCG AGG TAC CAG ATC TTA ATT AAT TAA GT

              NruI ———— BglII ———————

                 KpnI     STOPS

**D.  CSA**

               ↓ AluI                    ↓ RsaI

ATG ATG AGA AAA TTA GCT ATT -----//--------- TAG ATA AAG AAC ACA TCT TAG TTT GAG TTG TAC

MET MET ARG LYS LEU ALA ILE        END

1         7        410

**E.  #4148**

Pn promoter → ATG ATG CGT AAG CTC GCT ATT-----//--------- TAG ATA AAG AAC ACA TCT TAG TTT GAG TTG TCGAGGTACC

MET MET ARG LYS LEU ALA ILE        END

1         7        410

**F.  #3691**

Pn promoter → ATG CCC CCT ATT---//----------- TAG ATA AAG AAC ACA TCT TAG TTT GAG TTG TCGGGTACCAGATCTT

Met Pro Pro Ile        END

7        410

EP 0 329 257 A2

FIG. 4

# CIRCUMSPOROZOITE PROTEIN STRUCTURE: P. falciparum

SIGNAL

CHARGED REGION

REGION I

TETRAPEPTIDE REPEATS

CHARGED REGION

REGION II

CHARGED REGION

ANCHOR

75,000/68,000 dalton precursors CSA

60,000 dalton mature CSA

40   80   120   160   200   240   280   320   360   400   amino acid residues

EP 0 329 257 A2

FIG. 5